(19) Europäisches Patentamt
European Patent Office
Office européen des brevets

(11) Publication number: 0 322 747 B1

(12) EUROPEAN PATENT SPECIFICATION

(45) Date of publication of patent specification: 02.03.94 (51) Int. Cl.5: A61K 31/44

(21) Application number: 88121448.0

(22) Date of filing: 22.12.88

The file contains technical information submitted after the application was filed and not included in this specification

# (54) A brain neuron protecting agent containing a dihydropyridine compound.

(30) Priority: 29.12.87 JP 335582/87
17.05.88 JP 121619/88

(43) Date of publication of application:
05.07.89 Bulletin 89/27

(45) Publication of the grant of the patent:
02.03.94 Bulletin 94/09

(84) Designated Contracting States:
AT BE CH DE ES FR GB IT LI LU NL SE

(56) References cited:
FR-A- 2 438 654

Chemical and Pharmaceutical Bulletin, vol. 34, no. 8, Aug. 1986, pp. 3071-3078; A. Miyamae

Stroke, vol. 17, no. 2, March-April 1986, pp. 213-219; J. Grotta et al.

Japan J. of Pharmacology, vol. 38, no. 1, 1985, pp. 1-7, Kyoto, JP; A. Wauquier et al.

Stroke, vol. 20, no. 1, 1989, pp. 78-83, Dallas, US; T. Kuwaki et al.

(73) Proprietor: FUJISAWA PHARMACEUTICAL CO., LTD.
3, Doshomachi 4-chome
Higashi-ku
Osaka-shi Osaka 541(JP)

(72) Inventor: Kuwaki, Tomoyuki
5-25-16m Takanodai
Nerima-ku Tokyo 177(JP)
Inventor: Satoh, Hisashi
3-3-11, Koori
Ibaraki-shi Osaka 567(JP)
Inventor: Ono, Takaharu
6-4-2, Kofudai
Toyono-cho
Toyono-gun Osaka 563-01(JP)

(74) Representative: Türk, Gille, Hrabal, Leifert
Brucknerstrasse 20
D-40593 Düsseldorf (DE)

EP 0 322 747 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the use of a dihydropyridine compound of the formula:

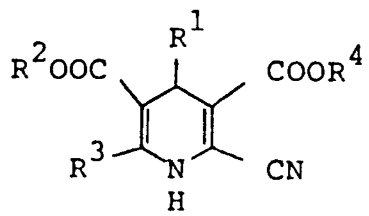

[I]

wherein $R^1$ is a nitrophenyl group and $R^2$, $R^3$ and $R^4$ are each same or different $C_1$-$C_6$ alkyl group or a pharmaceutically acceptable salt thereof, for the manufacture of a medicament for protecting brain neuron against cerebral ischemia due to cerebrovascular disease in mammal including human being. The dihydropyridine compound of the formula [I] which is used in this invention, is a known compound as described in British Patent No. 2,036,722. In regard to its pharmacologic actions, the compound has been found to be of value as an antianginal or antihypertensive agent thanks to its $Ca^{2+}$ antagonist activity or as a cerebral circulation improving agent or an antiarteriosclerotic agent and patents are pending in the name of the present applicant (Japanese Patent Application No. 161648/1987 and Japanese Patent Unexamined Publication No. 155327/1986).

It is an object of this invention to provide a medicament which inhibits by its $Ca^{2+}$ antagonistic activity the breakdown of cerebral neurocytoskeletal proteins due to intracranial ischemic disorder and more particularly to provide a brain neuron protecting agent predicated on the above-mentioned inhibitory activity.

The brain neuron protecting medicament according to this invention contains as an active ingredient a dihydropyridine compound of the formula [I] or a pharmaceutically acceptable salt thereof.

Referring to formula [I], the nitrophenyl group of $R^1$ includes 2-nitrophenyl, 3-nitrophenyl and 4-nitrophenyl and preferably is 3-nitrophenyl. The $C_1$-$C_6$ alkyl group of $R^2$, $R^3$ or $R^4$ is an alkyl group containing 1 to 6 carbon atoms such as methyl, ethyl, propyl, isopropyl, butyl, isobutyl, sec-butyl, pentyl, isopentyl, neopentyl, 1-methylbutyl, 2-methylbutyl and hexyl, and is preferably an alkyl group containing 1 to 4 carbon atoms. The most desirable example of $R^2$ is isopropyl and the most desirable example of $R^3$ and $R^4$ is methyl.

The pharmaceutically acceptable salt of dihydropyridine compound of formula [I] may be any nontoxic salt such as salts with the common inorganic bases such as alkali metals (for example, sodium, potassium, etc.), alkaline earth metals (for example, calcium, magnesium, etc.), ammonium and so on and salts with the common organic bases such as organic amines (for example, triethylamine, pyridine, picoline, ethanol amine, triethanolamine, dichlorohexylamine, N,N′-dibenzylethylenediamine, etc.).

While the aforementioned dihydropyridine compound [I] or pharmaceutically acceptable salt has been shown to be of use as an antianginal agent, antihypertensive agent, cerebral circulation improving agent or antiarteriosclerotic agent, further research has revealed that said compound or salt has a brain neuron protecting action as an action hitherto unknown to its pharmacology.

It has been confirmed that the dihydropyridine compound [I] has cerebrovasodilating activity, cerebral blood flow increasing activity and brain energy metabolism improving activity and is expected to be clinically effective against cerebral ischemia due to cerebrovascular disease. However, what matters ultimately in cerebral ischemic disorder is an impairment of the cerebral nerve cells and it was considered necessary to make explorations from this point of view. According to Yamashita et al. [Neurochemistry 24, 31-33 (1985)], when a transitory ischemic disorder was applied to the brain of a gerbil and, after recovery, the intracranial protein was fractionated and quantificated, it was found that only certain proteins (described hereinafter) associated with $Ca^{2+}$ had been decreased, that the above changes were observed in a very early stage following the onset of ischemia and that these changes last for a long time in contrast with an early improvement in brain energy metabolism, thus being consistent with histopathological findings. It is, thus, already clear that changes in certain proteins only are found in close time dependent with ischemic disorder. On the other hand, the studies done by the present inventors in regard to the pharmacological actions of dihydropyridine compound [I] revealed that this dihydropyridine compound [I] specifically inhibits the influx of extracellular $Ca^{2+}$ into the cerebral nerve cells at onset of ischemia and that, as a result, the breakdown of cerebral neurocytoskeletal proteins is inhibited to protect the nerve cells. Thus, as an

ischemic lesion develops in the brain, the $Ca^{2+}$ concentration in the cerebral nerve cells increase to thereby increase the activity of calpain (or CANP: calcium activated neutral protease), which presumably results in the breakdown of neurocytoskeletal proteins such as MAP-2 (microtube associated protein-2) and calspectin. While the above increase in $Ca^{2+}$ concentration is due to the influx of $Ca^{2+}$ from the extracellular environment, it has been found that dihydropyridine compound [I] inhibits the above breakdown of proteins by arresting the inflow of extracellular $Ca^{2+}$.

While the principle of such action of dihydropyridine compound [I] is that acting as a $Ca^{2+}$ antagonist, it inhibits the inflow of extracellular $Ca^{2+}$ into the cerebral nerve cells as mentioned above, further investigation by the present inventors revealed that nicardipine and nimodipine, which are also known as $Ca^{2+}$ antagonists, were exceedingly low in such pharmacologic actions. Thus, the substances heretofore called $Ca^{2+}$ antagonists are varying in chemical structure and have nothing in common and it is considered that even though they may be classed as $Ca^{2+}$ antagonists, these substances exhibit their respective pharmacologic actions according to their characteristic chemical structures.

The above-mentioned specific $Ca^{2+}$ antagonistic action of dihydropyridine compound [I] has been chiefly confirmed as a prophylactic effect on experimental ischemic lesions. Thus, as will be seen from the pharmacological tests to be described hereinafter, the prophylactic effect of compound [I] was investigated in two different settings, namely

(A) The compound was administered 1 hour before ischemic treatment and

(B) The compound was repeatedly administered not only 1 hour before ischemic treatment but also once a day for 4 consecutive days after the ischemic treatment. Based on results of these experiments, a further experiment was performed in the following setting: -

(C) The compound was not administered before ischemic treatment but administered for the first time after the ischemic treatment. It was found, as a result, that the above-mentioned specific $Ca^{2+}$ antagonistic action takes place even in the third setting (C) as well. Whereas the first-mentioned two settings were chiefly designed to demonstrate the prophylactic efficacy, the third setting (C) was intended to show the effectiveness for prognostic improvement after onset of an ischemic disorder (for example, cerebral apoplexy), and the above three experiments revealed that dihydropyridine compound [I] is useful for ischemic disorder, both prophylactically and therapeutically.

The brain neuron protecting agent according to this invention can be administered orally or otherwise to mammalian animals including man in the conventional dosage forms such as capsules, microcapsules, tablets, granules, powders, troches, pills, ointment, suppositories, injection, syrup and so on.

The brain neuron protecting agent of this invention can be manufactured by the established pharmaceutical procedure using the organic and/or inorganic carriers or vehicles which are commonly used in the art, for example excipients such as sucrose, starch, mannit, sorbit, lactose, glucose, cellulose, talc, calcium phosphate, calcium carbonate, etc.; binders such as cellulose, methylcellulose, hydroxymethylcellulose, polypropylpyrrolidone, gelatin, gum arabic, polyethylene glycol, sucrose, starch, etc.; disintegrating agents such as starch, carboxymethylcellulose, hydroxypropyl-starch, sodium hydrogen carbonate, calcium phosphate, calcium citrate, etc.; lubricating agents such as magnesium stearate, aerosil, talc, sodium laurylsulfate, etc.; corrigents such as citric acid, menthol, glycine, orange powder, etc.; preservatives such as sodium benzoate, sodium bisulfite, methylparaben, propylparaben, etc.; stabilizers such as citric acid, sodium citrate, acetic acid, etc.; suspending agents such as methylcellulose, polyvinylpyrrolidone, aluminum stearate, etc.; dispersing agents such as hydroxypropylmethylcellulose etc.; diluents such as water etc.; ointment bases such as cacao butter, white petrolatum, polyethylene glycol, etc., and so on.

The dosage of dihydropyridine compound [I] depends on the patient's body weight and/or age, the severity of disease and/or various other factors such as the method of administration. Usually, however, 0.1 to 1000 mg, and preferably 0.5 to 200 mg, per day is administered by the oral route. The effective dose per kilogram body weight is selected from the range of 0.001 to 20 mg and preferably 0.01 to 2 mg.

To substantiate the usefulness of the dihydropyridine compound [I] or pharmaceutically acceptable salt contained in the brain neuron protecting agent of this invention, pharmacological data on the compound are presented below.

Test compound

Isopropyl 6-cyano-5-methoxycarbonyl-2-methyl-4-(3-nitrophenyl)-1,4-dihydropyridine-3-carboxylate (hereinafter referred to as dihydropyridine compound A).

This compound was dissolved in polyethylene glycol 400 (PEG 400) for use.

Method

[A] Prophylactic effect of dihydropyridine compound A

(1) Breakdown of cytoskeletal proteins in a brain ischemia model

A gerbil was immobilized in supine position and median section was performed in the cervical region under local anesthesia. Then, the bilateral common carotid arteries were stripped off carefully not to injure the vagus and sympathetic nerves running in parallel, . Then, each common carotid artery was obstructed with a small clip and after an interval of 5, 10 or 15 minutes, the clips were removed. The animal was decapitated immediately, 4 days or 1 week after the removal of the clips, and the brain was isolated. Untreated animals served as controls and animals undergoing sham operation (involving the stripping procedure only) were also investigated. The isolated brain was sectioned under ice-cooling into the frontal cortex (upper 2/3, approximately corresponding to the 1st to the 5th layer, ~80 mg), striatum (~20 mg) and hippocampus (~50 mg) and each portion was homogenized in 4 times volume of (v/w) of 0.01 M phosphate buffer (pH 7.2). Each homogenate was centrifuged at 100,000 G for 30 minutes to obtain a supernatant. Equal parts of these supernatants were subjected to SDS-polyacrylamide gel (5%) electrophoresis and the proteins, namely MAP-2 (MW: 250-260K) calspectin (MW: 230-240K) and clathrin (MW: 180K), were quantified by measuring the absorbances at 560 nm with a densitometer (CS-930, Shimazu), and the proportion of each protein to the total water-soluble protein (exactly, the integral sum of the absorbance areas) was calculated. In addition, the relative percentage to the value of the untreated control group was also calculated.

(2) Effect of dihydropyridine compound A on the breakdown of cytoskeletal proteins ischemic

Two series of experiments were carried out. In the first series, dihydropyridine compound A [0, 0.01, 0.1 and 1 mg/kg] was administered subcutaneously one hour before occulsion and after 5 minutes of ischemia, reperfusion was carried out. One hour after the reperfusion, the animal was killed by decapitation and the brain was removed. In the second series, dihydropyridine compound A [0, 0.1, 1, and 10 mg/kg] was similarly administered subcutaneously one hour before the start of 5-minute ischemia and thereafter the same dose was subcutaneously administered once a day for 4 concesutive days (total: 5 times). One hour after the final administration (day 5), the animal was decapitated and the brain was isolated. The isolated brain was subjected to fractionation and electrophoresis as described above.

(3) Calcium-dependent breakdown of soluble cytoskeletal proteins

The whole brain of an untreated animal was homogenized and centrifuged and aliquots of the resulting supernatant was incubated at 37 °C for 60 minutes under the following 3 conditions viz. in the presence of 2 mM calcium chloride or 1.4 mM ethylene glycol-bis (β-aminoethyl ether)-N,N,N′,N′-tetraacetic acid (EGTA) or in the absence of any additive. The incubates were respectively subjected to electrophoresis and densitometry for quantification of proteins.

[B] Therapeutic effect of dihydropyridine compound A

A gerbil was immobilized in supine position and median section was performed in the cervical region under local anesthesia. Then, using care not to injure the parallel vagus and sympathetic nerves, the bilateral common carotid arteries were stripped off. Each common carotid artery was obstructed with a small clip for 5 minutes and the clip was removed. The animal was decapitated after 1 hour or 4 days and the brain was isolated. The animals with sham operation served as controls and untreated animals were also used. The isolated brain was treated and studied in the same manner as in Method (1).

The dosing schedule was as follows:

In one experiment, dihydropyridine compound A (0.1 mg/kg) was subcutaneously administered 0, 10 or 30 minutes after the 5-minute ischemia and the animal was decapitated 1 hour after completion of ischemia for isolation of the brain. In the other experiment, dihydropyridine compound A (0, 1 or 10 mg/kg) was subcutaneously administered 0, 10, 30 or 60 minutes after completion of ischemia and, then, further administered subcutaneously in the same dose once a day for the following 4 consecutive days (for a total of 5 doses). The animal was decapitated 1 hour after the last administration (day 5) and the brain was isolated. In both experiments, however, the vehicle group received the vehicle only immediately after

ischemia. The isolated brain was fractionated and subjected to electrophoresis in the described manner.

Results

[A] Prophylactic effect of dihydropyridine compound A

(1) Breakdown of cytoskeletal proteins in the brain ischemia model

Electrophoretically, MAP-2 was detected as 2 bands in the molecular weight range of 250-260 kilodaltons (K), calspectin as 3-4 bands in the range of 230-240 K, and clathrin as a single band at 180 K. These results were in complete agreement with the data reported by Nishimura, Yamashita et al. who performed immunoblotting with the respective antibodies. Under the conditions of the present study, the above three proteins were all that were found to undergo changes in concentration and a sum of these were no more than 5% of the total protein.

When the duration of ischemia was varied from 5 to 10 to 15 minutes and the respective protein concentration at 1 week after the ischemia was analyzed, it was found that in the case of 5-minute ischemia, only MAP-2 and calspectin decreased and there was no change in clathrin. In the case of 10-minute ischemia, three proteins decreased in the cortex and striatum, especially MAP-2 and calspectin. On the other hand, there were no changes in the hippocampus. In the case of 15-minute ischemia, MAP-2 and calspectin decreased in all brain sites but not clathrin. MAP-2 and calspectin tended to decrease markedly as the duration of ischemia increased but no clear decrease was observed in clathrin. The mortality rate was 0/5 (0%) for 5-minute ischemia, 8/17 (47%) for 10-minute ischemia and 31/33 (94%) for 15-minute ischemia, and most of the animals died within 24 hours after ischemia.

The duration of ischemia was fixed at 5 minutes and the respective protein were quantified immediately, 4 days or 1 week after ischemia. The 5-minute duration of ischemia was chosen because the 10-minute and longer durations of ischemia were considered unsuitable for the evaluation of effect in view of the mortality rates referred to above. Immediately after, 4 days after ischemia, each protein decreased in all brain areas, though there were few exceptions. After 1 week, only MAP-2 and calspectin had been decreased but they were in lesser degrees compared with those of immediately ischemia or 4 days after, furthermore clathrin showed substantially no decrease.

Effect of calcium or EGTA which is a calcium chelating agent, on the water-soluble brain fraction of normal animals, that is, the degradation of respective protein in vitro was examined. MAP-2 and calspectin were found to be markedly decreased in the presence of calcium, while clathrin showed little change. MAP-2 showed slight decreases even in the absence of calcium or EGTA. As MAP-2 is known to be a heat-resistant protein, and this slight decrease of MAP-2 may be due to the trace amount of endogenic calcium which was contained in the solutions.

(2) Effect on the ischemic breakdown of cytoskeletal proteins

The results of quantification of respective protein at 1 hour and 4 days after ischemia revealed that dihydropyridine compound A significantly inhibited the decrease in MAP-2 at all of the brain sites. The compound significantly inhibited decreases in calspectin in the hippocampus at one hour after ischemia and in the hippocampus and cortex 4 days after ischemia. The compound had no effect on decreases in clathrin. While the effective dose range was 0.01-10 mg/kg, there was no appreciable enhancement of effect at 1 mg/kg and higher doses and it appeared that the effect had reached a maximum within the range of 1 to 10 mg/kg. Decreases of the three proteins in the vehicle group tended to be greater in the striatum and hippocampus than in the cortex and the inhibitory effect of dihydropyridine compound A tended to be stronger in the striatum. Comparison of protein concentration at 1 hour after ischemia with those at 4 days after ischemia in the vehicle group showed that three proteins remained unchanged or tended to be slightly decreased. The breakdown of proteins was further in progress at 4 days, and the difference from the normal value was greater, and this suggested that the effects of dihydropyridine compound A would be examined easily at 4 days after ischemia. However the inhibitory effect of dihydropyridine compound A failed to show an overt difference between 1 hour after ischemia (single dosing) and after 4 days (5 repeated dosing).

The above experimental results may be summarized as follows. Whereas dihydropyridine compound A inhibited decreases in MAP-2 and calspectin which are presumed to be degraded by CANP, it had no effect on the decrease of clathrin which is independent on calcium. This finding not only suggests the prophylactic effect of dihydropyridine compound A against ischemic disorder of the brain but also suggests that the calcium influx into the nerve cells is actually occurring in ischemic states.

Comparison with reference substances

For a comparison of the action of dihydropyridine compound A with the actions of nimodipine and nicardipine which are conventional $Ca^{2+}$ antagonists, an experiment was performed according to the procedure set forth in Method (1). The results are summarized in Tables 1 to 3. As the vehicle, PEG-400 was used throughout. It is apparent from the tables that whereas the actions of nimodipine and nicardipine were extremely weak, only dihydropyridine compound A exhibited a significantly more potent action.

[B] Therapeutic effect of dihydropyridine compound A

The results of quantification of the respective protein at 1 hour after ischemia are shown in Table 4. At the dose of 1 mg/kg, dihydropyridine compound A which was administered immediately after ischemia significantly inhibited the decrease of MAP-2 in all the 3 brain sites and even when administered 10 minutes after ischemia, it significantly inhibited the decrease of MAP-2 in the hippocampus. Against the decrease of calspectin, the dihydropyridine compound A was effective only in the hippocampus and this effect was observed even when it was administered 30 minutes after ischemia. The dihydropyridine compound A had no effect on the decrease of clathrin.

The results of quantification of the respective protein at 4 days after ischemia are shown in Table 5. In the striatum, the decrease of MAP-2 was significantly inhibited in the 1 mg/kg/day and 10 mg/kg/day groups which received the first dosage immediately after ischemia or 10 minutes after ischemia and in the 10 mg/kg/day group which received the first dosage 30 minutes after ischemia. In the hippocampus, the decrease of MAP-2 was significantly inhibited in both 1 mg/kg/day and 10 mg/kg/day groups in which the administration was started immediately after to 60 minutes after ischemia.

Based on the above results, it is clear that the dihydropyridine compound A was effective against the decrease of MAP-2 in the hippocampus though the interval between ischemia and the start of administration was comparatively so long. On the other hand as to striatum and frontal cortex, it appeared preferable that the dihydropyridine compound A should be administered as early as possible.

Table 1 Effect on protein concentration at 1 hour after ischemia (% of normal value)

| Site of brain | Dosage (mg/kg) | n | MAP-2 | Calspectin | Clathrin |
|---|---|---|---|---|---|
| Cortex | | | | | |
| Sham operation control | | 9 | 84.9± 6.0 | 88.6± 4.8 | 102.4± 6.0 |
| Vehicle control | | 11 | 61.8± 1.6≠≠ | 84.9± 5.0 | 76.5± 5.5≠≠ |
| Dihydropyridine | 0.01 | 4 | 80.6±11.4 | 97.6± 8.9 | 80.5±11.4 |
| compound A | 0.1 | 5 | 77.6± 3.5 | 89.5± 3.5 | 77.5± 4.6 |
| | 1 | 6 | 97.3± 8.4 | 91.7± 7.8 | 79.9± 3.6 |
| Nimodipine | 0.01 | 5 | 55.7± 7.8 | 74.2± 8.5 | 81.9± 5.5 |
| | 0.1 | 5 | 61.8±10.6 | 67.2±13.6 | 81.8± 6.3 |
| | 1 | 5 | 65.3± 6.3 | 80.2± 6.0 | 85.9± 4.0 |
| Nicardipine | 0.01 | 5 | 67.8± 7.7 | 80.5± 5.1 | 69.7± 5.8 |
| | 0.1 | 5 | 78.1± 7.0 | 88.2± 4.5 | 66.4± 5.0 |
| | 1 | 5 | 85.6± 9.7* | 88.8± 5.3 | 67.2± 3.7 |
| Striatum | | | | | |
| Sham operation control | | 9 | 93.7±12.4 | 83.2± 8.0 | 102.5±14.1 |
| Vehicle control | | 11 | 31.2± 4.3≠≠ | 40.8± 6.4≠≠ | 45.3± 4.0≠≠ |
| Dihydropyridine | 0.01 | 4 | 63.7±17.3* | 60.1±14.8 | 41.3± 6.6 |
| compound A | 0.1 | 5 | 70.2±13.8** | 49.6± 7.1 | 57.7±18.5 |
| | 1 | 6 | 74.3± 5.7** | 62.7± 6.5 | 50.0± 7.8 |
| Nimodipine | 0.01 | 5 | 54.5± 5.9* | 51.8± 7.5 | 70.5±21.1 |
| | 0.1 | 5 | 53.9± 7.8* | 67.6±12.2 | 68.7±11.9 |
| | 1 | 5 | 56.8± 7.1* | 63.9±17.6 | 69.8±12.7 |
| Nicardipine | 0.01 | 5 | 53.5± 5.4* | 56.6± 4.4 | 53.1± 9.2 |
| | 0.1 | 5 | 56.7± 9.4* | 63.7± 5.8 | 48.3± 3.9 |
| | 1 | 5 | 55.0± 4.3* | 54.7± 6.0 | 64.5± 6.5 |
| Hippocampus | | | | | |
| Sham operation control | | 9 | 86.4± 5.0 | 86.6± 5.3 | 85.6±11.1 |
| Vehicle control | | 11 | 42.2± 2.2≠≠ | 62.3± 4.2≠≠ | 40.5± 4.0≠≠ |
| Dihydropyridine | 0.01 | 4 | 53.8± 7.5 | 79.9±12.6 | 50.3±19.7 |
| compound A | 0.1 | 5 | 56.9± 4.1 | 73.7± 7.3 | 44.9±12.4 |
| | 1 | 6 | 67.0± 6.6** | 93.3±11.6* | 42.7± 4.0 |
| Nimodipine | 0.01 | 5 | 41.7± 2.6 | 58.9± 4.6 | 52.1± 3.3 |
| | 0.1 | 5 | 44.5± 5.4 | 78.3±12.7 | 56.7± 7.1 |
| | 1 | 5 | 61.9± 9.9* | 84.5±18.3 | 61.6±12.2 |
| Nicardipine | 0.01 | 5 | 45.2± 6.2 | 78.5±11.8 | 53.0± 6.1 |
| | 0.1 | 5 | 46.7± 2.6 | 70.4± 5.3 | 49.0± 3.3 |
| | 1 | 5 | 50.5± 4.4 | 65.4± 9.2 | 56.9± 7.6 |

Each value represents the mean ± S.E.

≠,≠≠: P<0.05, 0.01 (compared with sham operation control group)

*,**: P<0.05, 0.01 (compared with vehicle control group)

Table 2 Effect on protein concentration at 4 days after ischemia (% of normal value

| Site of brain | Dosage (mg/kg) | n | MAP-2 | Calspectin | Clathrin |
|---|---|---|---|---|---|
| Cortex | | | | | |
| Sham operation control | | 5 | 87.2± 8.0 | 87.4± 3.4 | 94.3± 5.5 |
| Vehicle control | | 10 | 37.1± 2.6≠≠ | 55.6± 4.1≠≠ | 53.3± 3.5≠≠ |
| Dihydropyridine | 0.1 | 5 | 35.9± 5.0 | 65.9± 2.6 | 47.2± 0.8 |
| compound A | 1 | 4 | 70.1± 4.6** | 80.7± 7.1** | 49.1± 5.2 |
| | 10 | 5 | 65.3± [illegible].0** | 77.8± 5.3** | 47.2± 3.3 |
| Nimodipine | 0.1 | 5 | 46.2±12.0 | 45.3± 8.9 | 52.3± 9.5 |
| | 1 | 4 | 35.7±14.2 | 45.6±15.1 | 55.8± 8.5 |
| | 10 | 4 | 41.6±16.5 | 44.2±13.0 | 54.6± 5.7 |
| Nicardipine | 0.1 | 4 | 41.6± 5.6 | 54.7± 8.3 | 65.0± 6.6 |
| | 1 | 5 | 50.9± 6.3 | 51.9±10.5 | 55.3± 2.6 |
| | 10 | 5 | 55.6± 6.1* | 64.6± 8.4 | 54.8± 2.0 |
| Striatum | | | | | |
| Sham operation control | | 5 | 83.2± 7.0 | 81.2±15.0 | 90.2± 8.2 |
| Vehicle control | | 10 | 32.4± 3.3≠≠ | 42.5± 5.4≠ | 49.8± 5.7≠≠ |
| Dihydropyridine | 0.1 | 5 | 37.6± 5.4 | 36.8± 5.0 | 47.3± 3.9 |
| compound A | 1 | 4 | 71.7±11.9** | 55.5± 6.7 | 42.4± 2.9 |
| | 10 | 5 | 58.1± 4.1** | 56.4± 2.5 | 44.2± 5.7 |
| Nimodipine | 0.1 | 5 | 37.2± 7.3 | 46.6± 9.3 | 53.6±11.3 |
| | 1 | 4 | 65.2± 3.1** | 59.1± 4.6 | 57.9± 5.3 |
| | 10 | 4 | 67.8±10.2** | 58.9± 5.3 | 62.0±10.2 |
| Nicardipine | 0.1 | 4 | 44.8± 2.9 | 55.6± 7.3 | 51.7± 7.4 |
| | 1 | 5 | 51.0± 6.8** | 59.6± 4.6 | 59.9± 7.3 |
| | 10 | 5 | 57.0± 2.2** | 56.8± 6.4 | 55.4± 7.3 |
| Hippocampus | | | | | |
| Sham operation control | | 5 | 92.2±11.3 | 112.4± 4.2 | 90.6± 4.9 |
| Vehicle control | | 10 | 34.6± 2.9≠≠ | 54.8± 4.0≠≠ | 50.3± 5.3≠≠ |
| Dihydropyridine | 0.1 | 5 | 38.4± 6.4 | 64.4± 8.3 | 53.0± 6.1 |
| compound A | 1 | 4 | 65.3± 6.6** | 82.4± 4.3** | 52.8± 5.8 |
| | 10 | 5 | 72.3± 4.9** | 87.1± 4.3** | 54.8± 3.0 |
| Nimodipine | 0.1 | 5 | 53.6± 5.2 | 69.3±11.2 | 46.6± 4.2 |
| | 1 | 4 | 54.8± 9.4 | 62.6±14.5 | 51.2±10.7 |
| | 10 | 4 | 57.2±13.0 | 69.5± 6.5 | 64.6±15.0 |
| Nicardipine | 0.1 | 4 | 44.8± 4.5 | 57.6± 4.8 | 48.6± 8.9 |
| | 1 | 5 | 38.9± 6.4 | 59.5± 6.7 | 50.3± 5.2 |
| | 10 | 5 | 49.8± 4.3 | 61.2± 9.1 | 50.4± 4.9 |

Each value represents the mean ± S.E.

≠,≠≠: $P<0.05$, 0.01 (compared with sham operation control group)

*,**: $P<0.05$, 0.01 (compared with vehicle control group)

Table 3

| Effect on decreases in cytoskeletal proteins in a 5-minute ischemia-reperfusion gerbil model | | | | | |
|---|---|---|---|---|---|
| | | Frontal cortex | Striatum | Hippocampus | Total score |
| MAP-2 | Dihydropyridine compound A | +/+ | +/+ | +/+ | 3/3 (6) |
| | Nimodipine | -/- | +/+ | +/- | 2/1 (3) |
| | Nicardipine | +/+ | +/+ | -/- | 2/2 (4) |
| Calspectin | Dihydropyridine compound A | -/+ | -/- | +/+ | 1/2 (3) |
| | Nimodipine | -/- | -/- | -/- | 0/0 (0) |
| | Nicardipine | -/- | -/- | -/- | 0/0 (0) |
| Clathrin | Dihydropyridine compound A | -/- | -/- | -/- | 0/0 (0) |
| | Nimodipine | -/- | -/- | -/- | 0/0 (0) |
| | Nicardipine | -/- | -/- | -/- | 0/0 (0) |

+: Statistically significant

-: Not effective

Left : Dosed 1 hour before ischemia and killed by decapitation 1 hour after ischemia.

Right: Dosed 1 hour before ischemia and for 4 subsequent days and killed by decapitation 1 hour after the last dosing.

Table 4

Effect of dihydroyridine compound A (1 mg/kg group) on ischemic decreases in brain proteins (killed by decapitation 1 hour after 5-minute ischemia) [% of normal value]

| Site of brain | Time of administration (time in minutes after ischemia) | n | MAP-2 | Calspectin | Clathrin |
|---|---|---|---|---|---|
| Cortex | Sham operation control | 9 | 93.8± 6.6** | 91.4± 4.9** | 99.1± 5.8** |
| | Vehicle control | 5 | 47.9± 4.3 | 53.5± 6.8 | 50.1± 5.3 |
| | 0 | 5 | 63.1± 3.2* | 60.7± 7.6 | 52.8± 6.4 |
| | 10 | 5 | 60.4± 7.7 | 49.5±10.7 | 44.9± 7.7 |
| | 30 | 5 | 50.1± 2.7 | 50.5± 6.8 | 59.3± 9.8 |
| Striatum | Sham operation control | 9 | 108.4±14.3** | 100.8± 9.6** | 104.4±14.4** |
| | Vehicle control | 5 | 36.1± 4.8 | 37.6± 7.0 | 55.5± 8.2 |
| | 0 | 5 | 70.8± 9.7* | 52.5± 5.1 | 49.2± 4.0 |
| | 10 | 5 | 47.6± 7.9 | 49.6± 6.1 | 50.1± 5.1 |
| | 30 | 5 | 46.5± 6.5 | 41.7± 3.0 | 52.0± 3.9 |
| Hippocampus | Sham operation control | 9 | 95.2± 5.5** | 86.9± 5.4** | 86.2±11.2* |
| | Vehicle control | 5 | 35.0± 6.3 | 44.1± 6.1 | 37.3± 9.7 |
| | 0 | 5 | 69.1± 7.6* | 67.8± 6.6* | 44.0± 6.1 |
| | 10 | 5 | 77.7±11.3* | 71.8± 5.5* | 50.5± 7.3 |
| | 30 | 5 | 52.5± 7.3 | 61.5± 5.2* | 51.9± 9.5 |

Each value represents the mean ± S.E.

* : P<0.05 (compared with sham operation control group)

**: P<0.01 (compared with vehicle control group)

Table 5 Effect of dihydroyridine compound A (1, 10 mg/kg/day x 5) on ischemic decreases in brain proteins (killed by decapitation 5 days after 5-minute ischemia) [% of normal value]

| Site of brain | Time of administration (time in minutes after ischemia) | n | MAP-2 | Calspectin | Clathrin |
|---|---|---|---|---|---|
| | Sham operation control | 5 | 96.3± 8.8** | 90.2± 3.5** | 91.3± 5.4** |
| | Vehicle control | 5 | 50.8± 5.4 | 59.2± 6.0 | 49.3± 2.2 |
| Cortex | 0( 1 mg/kg/day) | 5 | 57.5± 7.7 | 62.6± 7.5 | 54.8± 4.1 |
| | 0(10 mg/kg/day) | 5 | 58.0± 3.5 | 75.3± 6.3 | 55.9± 3.4 |
| | 10( 1 mg/kg/day) | 5 | 50.0± 3.9 | 62.0± 6.8 | 54.5± 3.5 |
| | 10(10 mg/kg/day) | 3 | 75.9± 7.9 | 76.1±12.4 | 44.4± 6.1 |
| | 30( 1 mg/kg/day) | 5 | 57.3±10.5 | 61.7± 4.7 | 56.0± 4.5 |
| | 30(10 mg/kg/day) | 5 | 62.7± 8.6 | 68.6± 5.3 | 58.3± 6.6 |
| | 60( 1 mg/kg/day) | 5 | 61.1± 9.9 | 69.4± 6.0 | 57.0± 2.8 |
| | 60(10 mg/kg/day) | 5 | 60.0± 9.7 | 67.6± 7.7 | 51.9± 5.3 |
| | Sham operation control | 5 | 96.2± 8.1** | 98.3±18.2 | 91.9± 8.3 |
| | Vehicle control | 5 | 42.9± 3.6 | 55.3± 4.9 | 64.6± 7.6 |
| Striatum | 0( 1 mg/kg/day) | 5 | 67.3± 3.2** | 52.0± 3.6 | 64.5± 6.7 |
| | 0(10 mg/kg/day) | 5 | 66.3± 4.0** | 55.0± 3.6 | 65.5± 5.8 |
| | 10( 1 mg/kg/day) | 5 | 62.6± 6.3* | 64.4± 4.4 | 68.0± 5.9 |
| | 10(10 mg/kg/day) | 3 | 61.8± 1.8* | 56.7± 7.0 | 58.7± 4.9 |
| | 30( 1 mg/kg/day) | 5 | 58.3± 9.2 | 57.9± 3.3 | 65.0± 5.1 |
| | 30(10 mg/kg/day) | 5 | 61.6± 2.5** | 54.3± 7.5 | 60.7± 7.3 |
| | 60( 1 mg/kg/day) | 5 | 56.9± 9.5 | 48.3± 4.5 | 53.4± 4.4 |
| | 60(10 mg/kg/day) | 5 | 52.2± 4.5 | 49.6± 5.1 | 54.9± 5.1 |
| | Sham operation control | 5 | 101.5±12.4** | 112.8± 4.2** | 91.2± 5.0** |
| | Vehicle control | 5 | 37.9± 5.1 | 58.6± 3.8 | 57.4± 5.1 |
| Hippo- | 0( 1 mg/kg/day) | 5 | 82.1± 7.0** | 67.4± 3.4 | 61.2± 9.1 |
| campus | 0(10 mg/kg/day) | 5 | 71.2± 7.9* | 62.4± 3.8 | 56.8± 3.4 |
| | 10( 1 mg/kg/day) | 5 | 65.0± 3.2** | 67.0± 4.0 | 62.9± 3.8 |
| | 10(10 mg/kg/day) | 3 | 78.9±15.5* | 66.2± 9.3 | 62.6± 4.2 |
| | 30( 1 mg/kg/day) | 5 | 61.4± 6.4* | 57.4± 1.8 | 63.1± 4.6 |
| | 30(10 mg/kg/day) | 5 | 66.6± 4.1** | 66.8± 3.4 | 63.8± 5.0 |
| | 60( 1 mg/kg/day) | 5 | 59.5± 7.5* | 60.4± 3.9 | 56.9± 5.0 |
| | 60(10 mg/kg/day) | 5 | 61.2± 5.7* | 60.6± 4.8 | 60.3± 4.1 |

Each value represents the mean ± S.E.
* : P<0.05 (compared with sham operation control group)
**: P<0.01 (compared with vehicle control group)

[Examples]

Example 1

| | |
|---|---|
| Dihydropyridine compound A | 100 g |
| Hydroxypropylmethylcellulose | 500 g |

Dihydropyridine compound A was dissolved in absolute ethanol (5 ℓ), followed by addition of hydroxypropylmethylcellulose to give a suspension. The organic solvent was then removed under reduced pressure to give a solid dispersion.

Example 2

| | |
|---|---|
| Dihydropyridine compound A<br>Hydroxypropylmethylcellulose<br>Sucrose | 100 g<br>500 g<br>9.4 kg |

Sucrose was added to a suspension containing dihydropyridine compound A and hydroxypropylmethylcellulose in absolute ethanol (5 ℓ) and the mixture was stirred. The organic solvent was then removed under reduced pressure to give a solid dispersion. This composition was processed into fine granules in the routine manner.

Example 3

| | |
|---|---|
| Dihydropyridine compound A<br>Hydroxypropylmethylcellulose<br>Lactose<br>Hydroxypropylcellulose with a low degree of substitution<br>Magnesium stearate | 100 g<br>500 g<br>6.87 kg<br>1.5 kg<br>30 g |

To a suspension of dihydropyridine compound A and hydroxypropylmethylcellulose in absolute ethanol (5 ℓ) were added lactose and hydroxypropylcellulose with a low degree of substitution and the resulting mixture was stirred. The organic solvent was then removed under reduced pressure to give a solid dispersion. This composition was processed into granules in the routine manner and after addition of magnesium stearate, tablets were manufactured by the established pharmaceutical procedure. Each tablet contains 2 mg of dihydropyridine compound A.

Example 4

Each tablet manufactured in Example 3 was film-coated with a coating composition consisting of hydroxypropylmethylcellulose (5.1 mg), titanium dioxide (1.6 mg), polyethylene glycol 6000 (0.8 mg), talc (0.4 mg) and yellow iron oxide (0.1 mg) to give film-coated tablets containing 2 mg of dihydropyridine compound A per tablet.

**Claims**

**1.** Use of the dihydropyridine compound of the formula:

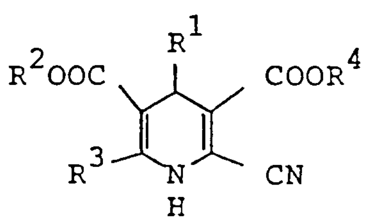

wherein $R^1$ is a nitrophenyl group and $R^2$, $R^3$ and $R^4$ are each same or different $C_1$-$C_6$ alkyl group or a pharmaceutically acceptable salt thereof, for the manufacture of a medicament for improving resistance of brain neurons in mammal including human being against damage resulting from ischemia.

**2.** Use claimed in Claim 1, wherein said dihydropyridine compound is isopropyl 6-cyano-5-methoxycarbonyl-2-methyl-4-(3-nitrophenyl)-1,4-dihydropyridine-3-carboxylate.

## Patentansprüche

1. Verwendung der Dihydropyridin-Verbindung der Formel:

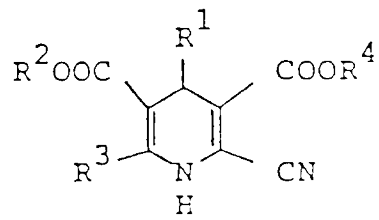

worin $R^1$ eine Nitrophenylgruppe ist und $R^2$, $R^3$ und $R^4$ jeweils eine gleiche oder verschiedene ($C_1$-$C_6$)-Alkylgruppe sind, oder eines pharmazeutisch verträgliches Salz davon zur Herstellung eines Medikamentes zur Verbesserung der Resistenz von Gehirnneuronen in Säugetieren, einschließlich Menschen, gegen eine Schädigung, die aus Ischämie resultiert.

2. Verwendung nach Anspruch 1, worin die genannte Dihydropyridin-Verbindung 6-Cyano-5-methoxycarbonyl-2-methyl-4-(3-nitrophenyl)-1,4-dihydropyridin-3-carbonsäureisopropylester ist.

## Revendications

1. Utilisation du composé de dihydropyridine de la formule :

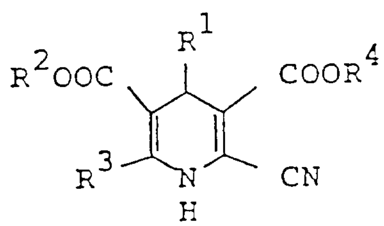

dans laquelle $R^1$ est un groupe nitrophényle et $R^2$, $R^3$ et $R^4$ sont chacun un groupe alkyle en $C_1$-$C_6$ identique ou différent, ou un de ses sels pharmaceutiquement acceptables, pour la fabrication d'un médicament afin d'améliorer la résistance des neurones du cerveau chez les mammifères dont l'être humain contre les endommagements résultant de l'ischémie.

2. Utilisation selon la revendication 1, dans laquelle le composé de dihydropyridine est le 6-cyano-5-méthoxycarbonyl-2-méthyl-4-(3-nitrophényl)-1,4-dihydropyridine-3-carboxylate d'isopropyle.